(19) **Europäisches Patentamt**

**European Patent Office**

**Office européen des brevets**

(11) Veröffentlichungsnummer: **0 259 617 B1**

(12) # EUROPÄISCHE PATENTSCHRIFT

(45) Veröffentlichungstag der Patentschrift: **04.03.92**

(21) Anmeldenummer: **87111426.0**

(22) Anmeldetag: **07.08.87**

(51) Int. Cl.5: **A61K 31/19**, A61K 31/22, A61K 35/78, A61K 9/02, //A61K9/06,A61K9/08,A61K9/12, A61K9/48,(A61K31/22,31:19, 31:12,31:215,31:11), (A61K31/19,31:215,31:12, 31:11),(A61K35/78,31:22,31:19, 31:215,31:12,31:11)

(54) **Verfahren und Präparat zur Inaktivierung von Viren.**

(30) Priorität: **08.09.86 LU 86573**

(43) Veröffentlichungstag der Anmeldung:
**16.03.88 Patentblatt 88/11**

(45) Bekanntmachung des Hinweises auf die Patenterteilung:
**04.03.92 Patentblatt 92/10**

(84) Benannte Vertragsstaaten:
**CH DE FR GB LI**

(56) Entgegenhaltungen:
**EP-A- 0 003 318**

(73) Patentinhaber: **CHIMICASA GMBH Wiesentalstrasse 81 CH-7000 Chur(CH)**

(72) Erfinder: **Lembke, Andreas, Prof. Dr.Dr. med. Eutiner Strasse 1 W-2420 Eutin-Sielbeck(DE)**
Erfinder: **Deininger, Rolf, Dr. Fürst-Pückler-Strasse 44 W-5000 Köln 41(DE)**

(74) Vertreter: **Hach, Hans Karl, Dr. Tarunstrasse 23 W-6950 Mosbach-Waldstadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

DIALOG-0215114, CANCERLIT, 1963-89/Juni, ICDB/81011413; M. KAWANISHI et al.: "Effect of short-chain fatty acids on Epstein-barr virus early and viral capsid antigen induction in P3HR-1 cells", & CANCER LETT; 11(2): 129-132, 1980

DIALOG 74135000, EMBASE (Exepta Medica) 1974-79, 0470040001925; K.A.J. HERNIMAN et al.: "The action of heat, chemicals and disinfectants on swine vesicular disease virus", & VET. REC. (ENGLAND), 1973, 93/24, p. 620-625

**Beschreibung**

Die Erfindung betrifft ein Verfahren zur Inaktivierung von Viren in Gegenwart lebender Zellen an Zellkulturen und ein pharmazeutisches Präparat zur Inaktivierung von Viren innerhalb lebender menschlicher oder tierischer Organismen.

Aus Herniman K.A.J., et al. THE VETERINARY RECORD, 93, 15. Dezember, 1973, pp. 620-625, ist es bekannt, daß 1/100 verdünnte Ameisensäure mit pH 2,5 eine zwar geringe Wirkung gegen Viren der Schwein-Vesikular Krankheit hat. Eine erheblich stärkere Wirkung wird mit einem Präparat aus 1/100 verdünnter Ameisensäure und 1/100 Benzolsulfonsäure und 1/1000 verdünntem Hederol mit pH 2,2 erzielt.

Aus EP-A-0 003 318 ist es bekannt, daß Schwarzer Pfeffer-Öl viruzide Wirkung hat.

Erste Aufgabe der Erfindung ist es, für ein Verfahren der eingangs genannten Art eine Wirksubstanz zur Verfügung zu stellen, die, jedenfalls in der für die viruzide Wirkung hinreichenden Dosierung, unschädlich ist für Zellkulturen, die mit den zu bekämpfenden Viren befallen sind.

Die Lösung dieser Aufgabe ist im Anspruch 1 gekennzeichnet.

Aufgrund eines synergistischen Effektes wird die angestrebte Wirkung erzielt in einem wenig sauren bis fast neutralen pH-Bereich.

Es empfiehlt sich, daß zur Neutralisation der eingesetzten Ameisensäure und des eingesetzten Ameisensäureester beziehungsweise Ameisensäureethylester die jeweils 0,5 - 1,0-fache, vorzugsweise 0,85-fache Menge an Natriumhydroxid eingesetzt ist.

Eine weitere Aufgabe der Erfindung ist es, ein Präparat der eingangs genannten Art so auszugestalten, daß es, jedenfalls in der für die viruzide Wirkung hinreichenden Dosierung, unschädlich ist für Zellkulturen und lebende menschliche oder tierische Organismen.

Die Lösung dieser Aufgabe ist im Anspruch 3 gekennzeichnet.

Eine pharmazeutische Injektionslösung ist vorzugsweise dadurch gekennzeichnet, daß die Wirkstoffe nur aus wasserlöslichen Bestandteilen bestehen und daß 1 mg (Milligramm) Wirkstoff in 0,03 ml (Milliliter) bis 0,4 ml physiologischer Lösung mit pH 5 bis pH 7, vorzugsweise pH 5,5, gelöst ist.

In manchen Fällen bestehen die Wirkstoffe aus wasserlöslichen und öligen Anteilen. In einem solchen Fall empfiehlt sich eine Ausgestaltung derart, daß wasserlösliche Wirkstoffe in Wasser gelöst sind und in öligen Wirkstoffen unter Zusatz von Erdnußöl und/oder mittelkettigen Triglyceriden mittels Sojalezithin emulgiert sind mit einem Einsatz von 1 ml Erdnußöl oder mittelkettigem Triglycerid auf 5 bis 10 ml in Wasser gelösten wasserlöslichen Wirkstoffen.

Eine pharmazeutische, einnehmbare Kapsel ist vorzugsweise dadurch gekennzeichnet, daß eine Kapselhülle vorgesehen ist, die eine Kapselfüllung vollständig umschließt, daß die Kapselfüllung aus 15 bis 110 mg, vorzugsweise 100 mg, Wirkstoff versetzt mit Hilfsstoff besteht, daB der Hilfsstoff aus Sojalezithin und/oder Rüböl und gegebenenfalls Natriumhydroxid besteht und daß die Kapselhülle aus Gelatine und Glycerin im Mischungsverhältnis 3 : 1 bis 2 : 1 besteht.

Ein pharmazeutischer Stift ist vorzugsweise dadurch gekennzeichnet, daß er aus einer formstabilen, aber durch Abstreichen abtragbaren, aus Hilfsstoffen gebildeten Trägermasse besteht, daß der Wirkstoff im Gewichtsverhältnis 1,0 : 100 bis 10 : 100, vorzugsweise 4 : 100, in die Hilfsstoffe eingemischt ist und daß die Hilfsstoffe aus Vaseline album und Paraffin im Mischungsverhältnis 1 : 1 bis 2 : 1 mit gegebenenfalls einem Zusatz von Natriumhydroxid bestehen.

Eine pharmazeutische Salbe ist vorzugsweise dadurch gekennzeichnet, daß die Wirkstoffe im Mischungsverhältnis 1 : 100 bis 10 : 100, vorzugsweise 4 : 100 bis 10 : 100, in eine durch die Hilfsstoffe gebildete, Salbenträgermasse eingemischt sind, daß die Salbenträgermasse aus Softisan ( = eingetragenes Warenzeichen), vermischt mit Neutralöl im Mischungsverhältnis 5 : 2 bis 5 : 3, vorzugsweise 5,3 : 2,2, besteht und daß gegebenenfalls Natriumhydroxid mit eingemischt ist.

Pharmazeutisches Spray ist vorzugsweise dadurch gekennzeichnet, daß die Wirkstoffe im Mischungsverhältnis 1,0 : 100 bis 10 : 100, vorzugsweise 1 : 100 bis 6 : 100, in physiologische Lösung pH 5,5 eingemischt sind.

Pharmazeutisches Spray kann mittels einer Dosierpumpe versprüht werden, es kann aber auch unter Treibgasdruck über ein Dosierventil versprüht werden. Als Treibgas empfiehlt sich Kohlendioxyd, unter Umständen auch Frigen[R].

Die Erfindung wird nun anhand einiger Beispiele näher erläutert.

Pharmazeutische Kapsel

Zusammensetzung gemäß nachfolgender Tabelle 1

| Inhaltsstoffe Kapselfüllung einer Kapsel | | Beispiele 1 | 2 |
|---|---|---|---|
| **Wirkstoffe** | | | |
| Ameisensäure | mg | 40 | -- |
| Ameisensäure-ethylester | mg | -- | -- |
| Tetrahydrofolsäure (= aktivierte Ameisensäure) | mg | -- | 20 |
| Schwarzer Pfeffer-Öl | mg | 100 | 100 |
| Zimtblüten-Öl | mg | 5 | -- |
| Cardamom-Öl | mg | 5 | -- |
| Linallylacetat | mg | 5 | -- |
| Summe der Wirkstoffe | mg | 155 | 120 |
| **Hilfsstoffe** | | | |
| Natriumhydroxid | mg | 34 | -- |
| Wasser | mg | 66,0 | -- |
| Sojalecithin | g | 0,5 | 0,5 |
| Kapselhülle einer Kapsel | | | |
| Gelatine | mg | 15 | 15 |
| Glycerin | mg | 10 | 10 |
| **Bemerkungen** Tagesdosis * der Kapseln | Stück | 3 | 3 |
| Tagesdosis * der Inhaltsstoffe | mg | 465 | 360 |

Pharmazeutischer Stift

Zusammensetzung gemäß nachfolgender Tabelle 2

| Inhaltsstoffe | | B e i s p i e l e | | |
|---|---|---|---|---|
| | | 3 | 4 | 5 |
| Wirkstoffe | | | | |
| Ameisensäure | mg | 1,0 | 1,0 | -- |
| Ameisensäure-ethylester | mg | -- | -- | -- |
| Tetrahydrofolsäure (= aktivierte Ameisensäure) | mg | -- | -- | 3,0 |
| Schwarzer Pfeffer-Öl | mg | 3,0 | 3,0 | 3,0 |
| Zimtaldehyd | mg | -- | 0,5 | -- |
| Zimtsäureethylester | mg | -- | 0,5 | -- |
| Summe der Wirkstoffe | mg | 4,0 | 5,0 | 6,0 |
| Hilfsstoffe | | | | |
| Natriumhydroxid | mg | 0,85 | 0,85 | -- |
| Wasser | mg | 18,0 | 18,0 | -- |
| Vaseline album | g | 40 | 40 | 40 |
| Paraffin | mg | 37,15 | 36,15 | 54 |
| Bemerkungen Tagesdosis * äußerlich | mg | 2 | 2 | 2 |
| Tagesdosis * der Inhaltsstoffe | mg | 80 | 200 | 240 |

Pharmazeutische Salbe

Zusammensetzung gemäß nachfolgender Tabelle 3

| Inhaltsstoffe | | Beispiele 6 | 7 |
|---|---|---|---|
| **Wirkstoffe** | | | |
| Ameisensäure | mg | 1 | -- |
| Ameisensäure-ethylester | mg | -- | -- |
| Tetrahydrofolsäure (= aktivierte Ameisensäure) | mg | -- | 2 |
| Schwarzer Pfeffer-Öl | mg | 3,0 | 3,0 |
| Zimtblüten-Öl | mg | -- | -- |
| Cardamom-Öl | mg | -- | -- |
| Linallylacetat | mg | -- | -- |
| Zimtaldehyd | mg | -- | -- |
| Zimtsäureethylester | mg | -- | -- |
| Carvon | mg | -- | -- |
| cis/trans Citral | mg | -- | -- |
| Summe der Wirkstoffe | mg | 4,0 | 5,0 |
| **Hilfsstoffe** | | | |
| Natriumhydroxid | mg | 0,85 | -- |
| Softisan ® | mg | 55 | 55 |
| Neutralöl | mg | 22 | 22 |
| Wasser | mg | 18 | 18 |
| **Bemerkungen** | | | |
| Tagesdosis * äußerlich | mg | 4000 | 4000 |
| Tagesdosis * der Inhaltsstoffe | mg | 160 | 200 |

Pharmazeutisches Spray

Zusammensetzung gemäß nachfolgender Tabelle 4

| Inhaltsstoffe | | Beispiele | |
|---|---|---|---|
| | | 8 | 9 |
| ============================================================ | | | |
| **Wirkstoffe** | | | |
| Ameisensäure | mg | -- | 1 |
| Ameisensäure-ethylester | mg | -- | -- |
| Tetrahydrofolsäure (= aktivierte Ameisensäure) | mg | 3 | -- |
| Schwarzer Pfeffer-Öl | mg | 3 | 3 |
| Zimtblüten-Öl | mg | -- | -- |
| Cardamom-Öl | mg | -- | -- |
| Linallylacetat | mg | -- | -- |
| Zimtsäureethylester | mg | -- | -- |
| ------------------------------------------------------------ | | | |
| Summe der Wirkstoffe | mg | 6 | 4 |
| **Hilfsstoffe** Natriumhydroxid | mg | -- | 0,85 |
| physiologische Lösung pH 5,5 | mg | -- | -- |
| Frigen als Treibmittel | mg | 94,0 | 95,15 |
| **Bemerkungen** Tagesdosis | ml | 1 | 1 |
| Tagesdosis * der Inhaltsstoffe | mg | 60 | 40 |

* Die Tagesdosis bezieht sich auf einen 70 kg schweren erwachsenen Menschen.

**Patentansprüche**

1. Verfahren zur Inaktivierung von Viren in Gegenwart lebender Zellen an Zellkulturen, dadurch gekennzeichnet,
   daß ein aus Wirkstoffen und Hilfsstoffen bestehendes Ameisensäurepräparat eingesetzt wird,
   daß die Wirkstoffe einen Anteil von 10 bis 90 Gew.-% vorzugsweise 25 Gew.-% an Ameisensäure und/oder Ameisensäureester, vorzugsweise Ameisensäureethylester, enthalten,

daß der Rest der Wirkstoffe zu mindest 75 Gew.-% aus Schwarzer Pfeffer-Öl besteht und

daß die Hilfsstoffe eine durch Lösungs- und/oder Emulgierungsmittel gebildete pharmazeutische Applikationsträgersubstanz und außerdem eine gegebenenfalls zur Neutralisierung der Wirkstoffe auf pH 5 bis pH 6,5 erforderliche ausreichende Menge an Lauge enthalten.

2. Verfahren nach Anspruch 1, dadurch gekennzeichnet,

daß zur Neutralisation der eingesetzten Ameisensäure und des eingesetzten Ameisensäureester beziehungsweise Ameisensäureethylester die jeweils 0,5 - 1,0-fache, vorzugsweise 0,85-fache Menge an Natriumhydroxid eingesetzt ist.

3. Pharmazeutisches Präparat zur Inaktivierung von Viren innerhalb lebender menschlicher oder tierischer Organismen, dadurch gekennzeichnet,

daß es aus Wirkstoffen und Hilfsstoffen besteht,

daß die Wirkstoffe einen Anteil von 10 bis 90 Gew.-% vorzugsweise 25 Gew.-% an Ameisensäure und/oder Ameisensäureester, vorzugsweise Ameisensäureethylester, enthalten,

daß der Rest der Wirkstoffe zu mindest 75 Gew.-% aus Schwarzer Pfeffer-Öl besteht und

daß die Hilfsstoffe eine durch Lösungs- und/oder Emulgierungsmittel gebildete pharmazeutische Applikationsträgersubstanz und außerdem eine gegebenenfalls zur Neutralisierung der Wirkstoffe auf pH 5 bis pH 7,0 erforderliche ausreichende Menge an Lauge enthalten.

4. Pharmazeutisches Präparat nach Anspruch 3, dadurch gekennzeichnet,

daß die Wirkstoffe zu 20 bis 30 Gew.-%, vorzugsweise 25 Gew.-%, aus Ameisensäure und/oder Ameisensäureester, vorzugsweise Ameisensäureethylester, bestehen und

daß der Rest der Wirkstoffe aus Schwarzer Pfeffer-Öl besteht.

5. Pharmazeutisches Präparat nach einem der Ansprüche 3 und 4, dadurch gekennzeichnet,

daß zur Neutralisation der eingesetzten Ameisensäure und des eingesetzten Ameisensäureester beziehungsweise Ameisensäureethylester die jeweils 0,5 - 1,0-fache, vorzugsweise 0,85-fache Menge an Natriumhydroxid eingesetzt ist.

6. Pharmazeutische Injektionslösung nach einem der Ansprüche 3 bis 5, dadurch gekennzeichnet,

daß die Wirkstoffe nur aus wasserlöslichen Bestandteilen bestehen und

daß 1 mg (Milligramm) Wirkstoff in 0,03 ml (Milliliter) bis 0,4 ml physiologischer Lösung mit pH 5 bis pH 7, vorzugsweise pH 5,5, gelöst ist.

7. Pharmazeutische Injektionslösung nach einem der Ansprüche 3 bis 5, dadurch gekennzeichnet,

daß wasserlösliche Wirkstoffe in Wasser gelöst sind und in öligen Wirkstoffen unter Zusatz von Erdnußöl und/oder mittelkettigen Triglyceriden mittels Sojalezithin emulgiert sind mit einem Einsatz von 1 ml Erdnußöl oder mittelkettigem Triglycerid auf 5 bis 10 ml in Wasser gelösten wasserlöslichen Wirkstoffen.

8. Pharmazeutische, einnehmbare Kapsel nach einem der Ansprüche 3 bis 5, dadurch gekennzeichnet,

daß eine Kapselhülle vorgesehen ist, die eine Kapselfüllung vollständig umschließt,

daß die Kapselfüllung aus 15 bis 110 mg, vorzugsweise 100 mg, Wirkstoff versetzt mit Hilfsstoff besteht,

daß der Hilfsstoff aus Sojalezithin und/oder Rüböl und gegebenenfalls Natriumhydroxid besteht und

daß die Kapselhülle aus Gelatine und Glycerin im Mischungsverhältnis 3 : 1 bis 2 : 1 besteht.

9. Pharmazeutischer Stift nach einem der Ansprüche 3 bis 5, dadurch gekennzeichnet,

daß er aus einer formstabilen, aber durch Abstreichen abtragbaren, aus Hilfsstoffen gebildeten Trägermasse besteht,

daß der Wirkstoff im Gewichtsverhältnis 1,0 : 100 bis 10 : 100, vorzugsweise 4 : 100, in die Hilfsstoffe eingemischt ist und

daß die Hilfsstoffe aus Vaseline album und Paraffin im Mischungsverhältnis 1 : 1 bis 2 : 1 mit gegebenenfalls einem Zusatz von Natriumhydroxid bestehen.

10. Pharmazeutische Salbe nach einem der Ansprüche 3 bis 5, dadurch gekennzeichnet,

daß die Wirkstoffe im Mischungsverhältnis 1 : 100 bis 10 : 100 in eine durch die Hilfsstoffe gebildete Salbenträgermasse eingemischt sind,

daß die Salbenträgermasse aus Softisan®, vermischt mit Neutralöl im Mischungsverhältnis 5 : 2 bis 5 : 3, vorzugsweise 5,3 : 2,2, besteht und

daß gegebenenfalls Natriumhydroxid mit eingemischt ist.

11. Pharmazeutisches Spray nach einem der Ansprüche 3 bis 5, dadurch gekennzeichnet,

daß die Wirkstoffe im Mischungsverhältnis 1,0 : 100 bis 10 : 100, vorzugsweise 1 : 100 bis 6 : 100, in physiologische Lösung pH 5,5 eingemischt sind.

**Claims**

1. Process for the inactivation of viruses in the presence of living cells on cell cultures, characterized in that a formic acid preparation comprising active agents and adjuvants is used, that the active agents contain 10 to 90 and preferably 25% weight of formic acid and/or formate, preferably ethyl formate, that the residue of the active agents comprises at least 75% weight black pepper oil and that the adjuvants contain a pharmaceutical application carrier substance formed solvents and/or emulsifiers and also a base quantity which be necessary for neutralizing the active agents to pH 5 to 6.5.

2. Process according to claim 1, characterized in that for neutralizing the formic acid and formate or ethyl formate used, 0.5 to 1.0 and preferably 0.85 times the sodium hydroxide quantity is used.

3. Pharmaceutical preparation for inactivating viruses within living human or animal organisms, characterized in that it comprises active agents and adjuvants, that the active agents contain 10 to 90 and preferably 25% by weight of formic acid and/or formate, preferably ethyl formate, that the remainder of the active agents comprises at least 75% by weight black peeper oil and that the adjuvants contain a pharmaceutical application carrier substance formed by solvents and/or emulsifiers and optionally an adequate base quantity necessary for neutralizing the active agents to pH 5 to 7.0.

4. Pharmaceutical preparation according to claim 3, characterized in that the active agents comprise 20 to 30 and preferably 25% by weight formic acid and/or formate, preferably ethyl formate and that the remainder of the active agents comprises black pepper oil.

5. Pharmaceutical preparation according to one of the claims 3 and 4, characterized in that for neutralizing the formic acid or formate/ethyl formate used, in each case 0.5 to 1.0 and preferably 0.85 times the sodium hydroxide quantity is used.

6. Pharmaceutical injection solution according to one of the clams 3 to 5, characterized in that the active agents only comprise water-soluble constituents and that 1 mg of active agent is dissolved in 0.03 to 0.4 ml of physiological solution with pH 5 to 7 and preferably pH 5.5.

7. Pharmaceutical injection solution according to one of the claims 3 to 5, characterized in that water-soluble active agents are dissolved in water and emulsified in oily active agents, accompanied by the addition of peanut oil and/or medium-chain triglycerides by means of soybean lecithin with a use of 1ml of peanut oil or medium-chain triglyceride for 5 to 10 ml of water-dissolved, water-soluble active agents.

8. Pharmaceutically injectable capsule according to one of the claims 3 to 5, characterized in that a capsule case is provided, which completely surrounds a capsule filling, that the capsule filling comprises 15 to 110 mg and preferably 100 mg of active agent mixed with adjuvant, that the adjuvant comprises soybean lecithin and/or rape oil and optionally sodium hydroxide and that the capsule case comprises gelatin and glycerin in a mixing ratio of 3:1 to 2:1.

9. Pharmaceutical stick according to one of the claims 3 to 5, characterized in that it comprises a dimensionally stable carrier mass formed by adjuvants and which can be removed by scraping off, that the active agent is mixed into the adjuvant in a weight ratio of 1.0:100 to 10:100 and preferably 4:100 and that the adjuvants comprise vaseline album and paraffin in a mixing ratio of 1:1 to 2:1 with optionally a sodium hydroxide addition.

10. Pharmaceutical ointment according to one of the claims 3 to 5, characterized in that the active agents are mixed into an ointment carrier mass formed by the adjuvants in a mixing ratio of 1:100 to 10:100, that the ointment carrier mass comprises Softisan, mixed with neutral oil in a mixing ratio of 5:2 to 5:3, preferably 5.3:2.2 and that optionally sodium hydroxide is also mixed in.

**11.** Pharmaceutical spray according to one of the claim 3 to 5, characterized in that the active agents are mixed in a physiological solution at pH 5.5 in a mixing ratio of 1.0:100 to 10:100 and preferably 1:100 to 6:100.

**Revendications**

**1.** Procédé pour inactiver des virus en présence de cellules vivantes de cultures de cellules,caractérisé en ce qu'on utilise une préparation d'acide formique consistant en des substances actives et des adjuvants; en ce que les substances actives contiennent une proportion de 10 à 90 % en poids , avantageusement 25 % en poids,d'acide formique et/ou d'un ester d'acide formique , avantageusement du formiate d'éthyle; en ce que le reste des substances actives consiste pour au moins 75 % en poids en de l'essence de poivre gris,et en ce que les adjuvants contiennent une substance support d'application pharmaceutique,formée par un solvant et/ou un émulsifiant et,en outre, une quantité de lessive alcaline nécessaire éventuellement et suffisante pour neutraliser à pH 5 à pH 6,5 les substances actives .

**2.** Procédé selon la revendication 1 , caractérisé en ce que , pour neutraliser l'acide formique utilisé et l'ester d'acide formique ou le formiate d'éthyle utilisé , on utilise à chaque fois 0,5 à 1,0 fois , avantageusement 0,85 fois la quantité d'hydroxyde de sodium correspondante.

**3.** Préparation pharmaceutique pour inactiver des virus à l'intérieur d'organismes humains ou animaux vivants ,caractérisée en ce qu'elle consiste en des substances actives et en des adjuvants; en ce que les substances actives contiennent une proportion de 10 à 90 % en poids ,avantageusement de 25 % en poids d'acide formique et/ou d'un ester d'acide formique , avantageusement le formiate d'éthyle; en ce que le reste des substances actives consiste pour au moins 75 % en poids en de l'essence de poivre gris ; et en ce que les adjuvants contiennent une substance support d'application pharmaceutique,formée par un solvant et/ou un émulsifiant,et en outre,éventuellemnt une quantité de lessive alcaline nécessaire et suffisante pour neutraliser à pH 5 à pH 7,0 les substances actives .

**4.** Préparation pharmaceutique selon la revendication 3 , caractérisée en ce que les substances actives consistent pour 20 à 30 % en poids, avantageusement 25 % en poids , d'acide formique et/ou d'un ester d'acide formique , avantageusement le formiate d'éthyle, et en ce que le reste des substances actives consiste en de l'essence de poivre gris .

**5.** Préparation pharmaceutique selon l'une des revendications 3 et 4 , caractérisée en ce que , pour neutraliser l'acide formique utilisé et l'ester d'acide formique ou le formiate d'éthyle utilisé , on utilise à chaque fois une quantité d'hydroxyde de sodium représentant 0,5 à 1,0 fois , avantageusement 0,85 fois la quantité d'acide et d'ester ou de formiate.

**6.** Solution pharmaceutique pour injection selon l'une des revendications 3 à 5 , caractérisée en ce que les substances actives ne consistent qu'en des constituants hydrosolubles,et en ce que 1 mg (milligramme) de substance active est dissous dans 0,03 ml (millilitre) à 0,4 ml de solution physiologique à pH 5 à pH 7 ,avantageusement à pH 5,5 .

**7.** Préparation pharmaceutique pour injection selon l'une des revendications 3 à 5 , caractérisée en ce que les substances actives hydrosolubles sont dissoutes dans de l'eau et sont émulsifiées à l'aide de lécithine de soja dans des substances actives huileuses, avec addition d'huile d'arachide et/ou de triglycérides à chaines de longueur moyenne,avec addition de 1 ml d'huile d'arachide ou de triglycéride à longueur moyenne de chaine pour 5 à 10 ml de substances actives hydrosolubles dissoutes dans de l'eau .

**8.** Capsule ou gélule pharmaceutique ingérable selon l'une des revendications 3 à 5 , caractérisée en ce qu'on prévoit une enveloppe de capsule entourant complètement un contenu de capsule;en ce que le contenu remplissant une capsule consiste en 15 à 110 mg,avantageusement 100 mg de substance active additionnée d'un adjuvant; en ce que l'adjuvant consiste en de la lécithine de soja et/ou en de l'huile de colza et éventuellement en de l'hydroxyde de sodium,et en ce que l'enveloppe de capsule consiste en gélatine et glycérol selon un rapport de mélange de 3:1 à 2:1 .

9. Crayon pharmaceutique selon l'une des revendications 3 à 5 , caractérisé en ce qu'il consiste en une masse porteuse formée d'adjuvants, de forme stable , mais pouvant être transférée par raclage; en ce que la substance active est incorporée selon un rapport pondéral de 1,0 : 100 à 10:100, avantageusement 4:100 ,aux adjuvants;et en ce que les adjuvants consistent en vaseline blanche et paraffine selon un rapport de mélange de 1:1 à 2:1 ,avec éventuellement une addition d'hydroxyde de sodium .

10. Pommade pharmaceutique selon l'une des revendications 3 à 5 , caractérisée en ce que les substances actives sont incorporées selon un rapport de mélange de 1:100 à 10:100 dans une masse de support ou base de pommade formée par les adjuvants;en ce que la masse de support ou de base de pommade consiste en "Softisan"$^R$ en mélange avec de l'huile neutre, selon un rapport de mélange de 5:2 à 5:3 ,avantageusement 5,3:2,2,et en ce qu'éventuellement de l'hydroxyde de sodium est aussi incorporé.

11. Produit pharmaceutique à pulvériser ,selon l'une des revendications 3 à 5 ,caractérisé en ce que les substances actives sont incorporées selon un rapport de mélange de 1,0:100 à 10:100,avantageusement 1:100 à 6:100, dans une solution physiologique à pH 5,5.